Europäisches Patentamt

European Patent Office

Office européen des brevets

(11) Publication number : **0 470 568 B1**

# EUROPEAN PATENT SPECIFICATION

(45) Date of publication of patent specification :
**22.06.94 Bulletin 94/25**

(51) Int. Cl.[5] : **A61K 31/16, A61K 9/02**

(21) Application number : **91113172.0**

(22) Date of filing : **06.08.91**

(54) **15-Deoxyspergualin suppository.**

(30) Priority : **10.08.90 JP 213505/90**

(43) Date of publication of application :
**12.02.92 Bulletin 92/07**

(45) Publication of the grant of the patent :
**22.06.94 Bulletin 94/25**

(84) Designated Contracting States :
**DE ES FR GB IT**

(56) References cited :
**DATABASE WPIL, accession no. 90-056185
[08], Derwent Publications Ltd, London, GB
DATABASE WPIL, accession no. 90-055509
[08], Derwent Publications Ltd, London, GB**

(73) Proprietor : **NIPPON KAYAKU KABUSHIKI
KAISHA
11-2, Fujimi 1-chome
Chiyoda-ku
Tokyo 102 (JP)**
Proprietor : **TAKARA SHUZO CO. LTD.
609 Takenakacho
Fushimi-ku Kyoto (JP)**

(72) Inventor : **Hiraga, Hironobu
1090-B-423, Kamiochiai
Yono-shi (JP)**
Inventor : **Okuma, Takaaki
1090-C-241, Kamiochiai
Yono-shi (JP)**
Inventor : **Umeda, Yoshihisa
27-21, Jinryo-2-chome
Otsu-shi (JP)**

(74) Representative : **Türk, Dietmar, Dr. rer. nat.
Türk, Gille, Hrabal, Leifert
Patentanwälte
Brucknerstrasse 20
D-40593 Düsseldorf (DE)**

EP 0 470 568 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

Spergualin is an antibiotic having anticancer activity which was found by Umezawa et al. (U.S. Patent 4,416,899). Many derivatives and analogs of spergualin having similar activity are disclosed in U.S. Patent 4,518,532, U.S. Patent 4,529,549, U.S. Patent 4,556,735 and Japanese Patent Laid-open 62-48660 whereas derivatives having immunosuppressive activity are disclosed in U.S. Patent 4,851,446.

Of these compounds, 15-deoxyspergualin [i.e. N-[4-(3-aminopropyl)aminobutyl]-2-(7-guanidinoheptaneamido)-2-hydroxyethaneamide)] is usable as an immunosuppressor. As preparations of spergualins, injectable preparations (U.S. Patent 4,876,244) and oral preparations for increasing the oral bioavailability by using simple or complex fats have been proposed (Japanese Patent Laid-open 2-9816 and ibid. 2-11514).

15-Deoxyspergualin is now being developed as an immunosuppressor. For the prophylaxis or therapy of the diseases wherein an immunosuppressor is needed continuous administration is required to some extent. Accordingly, it is desired to develop preparations other than injectable preparations capable of giving a high plasma concentration of the drug by easy administration at low dose. However, 15-deoxyspergualin hydrochloride shows insufficient bioavailability even when it is administered orally as mentioned above, and it is difficult to obtain a plasma concentration of the drug sufficient to impart an immunosuppresive effect by an administration route other than injection.

In addition, 15-deoxyspergualin hydrochloride is hygroscopic and has a poor stability, and therefore it was difficult to obtain preparations of this drug having good storage stability.

An object of the present invention is to provide a 15-deoxyspergualin preparation to solve the above-mentioned problems.

The present invention relates to a novel suppository containing 15-deoxyspergualin trihydrochloride β-form crystals and lipophilic base which is solid at ambient temperature, which can be used as an anticancer agent or an immunosuppressor.

As a result of the various researches, the present inventors have found that a powder or an aqueous syrup of known 15-deoxyspergualin trihydrochloride (hereinafter referred to as "15-deoxyspergualin hydrochloride") can be crystallized in the presence of a mixture of water and a water miscible solvent, and that the resulting crystals (hereinafter referred to as "β-form crystals") can be mixed with a lipophilic base which is solid at an ambient temperature to give a suppository, which imparts high plasma concentration of 15-deoxyspergualin when administered and has a good storage stability.

For formulation of the suppository of the present invention, one or a mixture of two or more lipophilic bases, each of which is solid at normal ambient temperature, are melted by heating, and 15-deoxyspergualin hydrochloride β-form crystals are added thereto, and homogenized. The mixture is filled into suppository containers or gelatin capsule shells and cooled to give suppositories.

15-Deoxyspergualin hydrochloride β-form crystals are used in an amount of about 1 - 40% (all percentages are given "by weight"), preferably 3 - 25%, more preferably 10 - 20% based on the total weight of the preparation. The amount of base is 60 - 99%, preferably 75 - 97%, and more preferably 80 - 90%. For various purposes, other additives can be added to the preparation of the present invention. The amount of the additives is 0 - 0.3 part (all parts are "part by weight") relative to 1 part of the mixture of 15-deoxyspergualin hydrochloride β-form crystals and the base. The amount of the base is not less than 3 parts, preferably 4 - 100 parts relative to 1 part of the β-form crystals.

β-Form crystals of 15-deoxyspergualin hydrochloride used in the present invention is prepared as follows: To about 100 parts of a powder of 15-deoxyspergualin hydrochloride obtained by a prior art method is added 500 parts of 95% ethanol, and the mixture is thoroughly mixed and allowed to stand at 5°C for 3 days, and the resulting crystals are removed by suction filtration to give the β-form crystals.

Table 1 shows an X-ray diffraction pattern of β-form crystals of 15-deoxyspergualin hydrochloride used in the present invention which is measured by means of an X-ray diffraction apparatus (XD-3A, Shimadzu Co.) equipped with a Cu X-ray apparatus using graphite monochrometer and with a scintillation counter.

| $d(10^{-10}m(\overset{\circ}{A}))$ | $I/I_1$ | $d(10^{-10}m(\overset{\circ}{A}))$ | $I/I_1$ |
|---|---|---|---|
| 15.77 | 0.32 | 4.14 | 0.70 |
| 10.91 | 0.42 | 3.66 | 1.00 |
| 4.88 | 0.39 | 3.31 | 0.52 |
| 4.67 | 0.53 | 3.21 | 0.52 |

(Electric power Cu: Graphite monochrometer, 40 KV-30 mA, $\lambda = 1.54051$)

The lipophilic base which is solid at ambient temperature for use in the present invention includes medium chain fatty acid triglycerides which are solid at an ambient temperature (e.g. capric acid triglyceride); and oil and fat suppository bases (e.g. cacao butter, laurine butter, cinnamon butter, hydrogenated oil, semi-synthetic glycerides, and the like). They can be used alone or in combination of two or more of them.

Examples of the lipophilic base which is solid at normal ambient temperature are triglycerides of $C_8$ - $C_{18}$ saturated fatty acids, which preferably contain at least 50% of $C_8$ - $C_{14}$ saturated fatty acids. The fatty acids which constitute the triglycerides preferably contain one or more members selected from $C_8$, $C_{10}$, $C_{12}$ and $C_{14}$ saturated fatty acids.

The fatty acids attached to glycerin preferably contain 32 to 60%, preferably 44 to 57% of a $C_{12}$ saturated fatty acid; 12 to 25%, preferably 17 to 23% of a $C_{14}$ saturated fatty acid; 9 to 20%, preferably 10 to 14% of a $C_{16}$ saturated fatty acid; 9 to 32%, preferably 10 to 22% of a $C_{18}$ saturated fatty acid; and 0 to 4%, preferably 0 to 2% of other fatty-acids based on the total weight of the fatty acids.

The suppository of the present invention may be a capsule for rectal use which is prepared by molding suppositories by use of gelatin capsule shells and filling a mixture of the medicine (solution or solid) and the base therein.

Besides the above components (15-deoxyspergualin and the suppository base), additives may be added to the suppository of the present invention, for example, viscosity modifying agents such as microcrystalline wax and microcrystalline cellulose; substances for reducing rectal irritation such as fractionated coconut oils; a small amount of lecithin for prevention of blooming of fats. The amount of these additives is 0 - 30% based on the total weight of the drug and the suppository base.

The present invention is illustrated by the following examples. 15-Deoxyspergualin is abbreviated as "DSG".

Example 1

(Formulation)

```
β-form of DSG              1 g

Pharmasol H-15*           26 g

* Nippon Oil & Fats Co., LTD.
```

The above components are for 20 suppositories.

Pharmasol H-15 (proportion of fatty acids: about 54% of a $C_{12}$ fatty acid; about 22% of a $C_{14}$ fatty acid; about 12% of a $C_{16}$ fatty acid; and about 12% of a $C_{18}$ fatty acid) in a beaker was melted by heating on a water bath. Heating was stopped and 1 g of DSG was added at 40°C to the solution under stirring and the components were homogenized under sufficient mixing. The mixture was poured into suppository molds (suppository containers), cooled at room temperature then solidified by cooling in a refrigerator to give suppositories.

Example 2

(Formulation)

```
        β-form of DSG                        1 g

        Panacete 1000*                      26 g

            * triglyceride of capric acid (made by

    Nippon Oil & Fats Oo., LTD).
```

The above components are for 20 suppositories.
In a similar manner to that of Example 1, suppositories were obtained.

Example 3

(Formulation)

β-form of DSG        5 g
Pharmasol H-15      35 g
The above components are for 20 suppositories.
In a similar manner to that of Example 1, suppositories were obtained.

Reference Example 1

A powder of DSG was dissolved in water to give a solution of 10 mg of DSG/ml.

Reference Example 2

50 mg of a powder of DSG and 50 mg of Panacete 1000 were mixed and filled into No. 3 capsules (the Pharmacopoeia of Japan).

Reference Example 3

(Formulation)

```
        Powder of DSG                        1 g

        Macrogol* 1500                      18 g

        Macrogol* 4000                       8 g

        (* Trade name,  polyethylenglycol)
```

The above components are for 20 suppositories.
Macrogol 1500 and Macrogol 4000 were taken into a beaker, and melted on a warmed water bath. DSG was added little by little to the solution with stirring and homogenized with enough mixing. The mixture was filled into suppository molds, solidified by cooling to give suppositories.

Test Example 1

(1) Test Method

Suppositories prepared in Examples 1 and 2 were each rectally administered to male beagles weighing 9.0 - 11.0 kg (3 animals were used for each group). As control, 5 ml of a solution obtained in Reference Example 1 was administered orally or rectally, a capsule obtained in Reference Example 2 was administered orally, and the suppository of Reference Example 3 was administered rectally. In all cases, doses of DSG were 5 mg/kg. Animals were fasted from the day before the beginning of the test to the conclusion of the test. Each about 5 mg of blood samples was collected into a heparin treated syringe at 0.5, 1, 2, 4 and 8 hours after administration, and centrifuged to separate the serum.

To 1 ml of the serum was added 1 μg of N-[4-(3-aminopropyl)aminobutyl]-2-(7-guanidinooctanamido)-2-hydroxyethanamide as an internal standard, and the mixture was diluted with 5 ml of distilled water and injected into CM-Sephadex® C-25 column [$Na^+$ type (Pharmacia Fine Chemical Co.) 5 cm x 0.5 cm φ]. The column was washed with 10 ml of 0.3 M aqueous sodium chloride solution, and then DSG and the internal standard were eluted with 10 ml of 0.5 M aqueous sodium chloride solution. After desalting by using Sep-Pak $C_{18}$ cartridge, the mixture was eluted with methanol, and the solvent was evaporated under reduced pressure.

The residue was dissolved in 250 μl of distilled water, and 100 μl of the solution was injected in a high performance liquid chromatograph (HPLC), treated with o-phthalaldehyde (OPA) to give its derivative (so-called postcolumn derivatization), which was determined by HPLC with fluorescence detector.

| | |
|---|---|
| HPLC: | Shimadzu liquid chromatography apparatus |
| Column: | Cosmosil $5C_{18}P$ (15 cm x 4.6 mmφ) |
| Column temperature: | 25°C |
| Detection wavelength: | excitation wavelength 335 nm, emission wavelength 435 nm. |
| Mobile phase: | 5mM sodium heptanesulfonate was dissolved in 10 mM phosphate buffer (pH 3.0) - acetonitrile (80:20). |
| Flow rate: | mobile phase 1.0 ml/min. OPA reagent 0.5 ml/min. |

(2) Test Results

## Table 2

## Pharmacokinetic Parameter

| Sample | $C_{max}$ (μg/ml) | AUC 0-8h (μg h/ml) |
|---|---|---|
| Example 1 | 1.126 | 2.310 |
| Example 2 | 0.890 | 1.396 |
| Control 1 (Oral administration of the aqueous solution) | 0.318 | 0.775 |
| Control 1 (Rectal administration of the aqueous solution) | 0.126 | 0.144 |
| Control 2 (Oral administration of the capsule) | 0.225 | 0.541 |
| Control 3 (Rectal administration | 0.217 | 0.320 |

$C_{max}$:      The maximum plasma concentration

AUC 0-8h :     Area under the plasma concentration - time curve (0 $\sim$ 8 hours)

Test Example 2

Stability (severe Test)

(Test Method)

To 50 mg of exactly weighed β-form crystals of DSG (or a lyophilized powder of DSG) in a hermetic vial was added 1.25 g of Witepsol® (proportion of fatty acids: about 54% of a $C_{12}$ fatty acid; about 22% of a $C_{14}$ fatty acid; about 12% of a $C_{16}$ fatty acid; about 12% of a $C_{18}$ fatty acid) which was melted by heating on a water bath, and then the mixture was thoroughly mixed. After being tightly closed, the mixture was kept at 50°C for 2 weeks or at 35°C for 4 weeks.

The amount of DGS extracted from the base was measured according to a HPLC technique.

## Table 3     Remaining ratio of DSG in the suppository base

| Sample | Remaining ratio of DSG | |
|---|---|---|
| | 35 °C, 4w | 50 °C, 2w |
| β-form crystals of DSG | 98.7% | 101.8% |
| Lyophilized powder of DSG | 95.8% | 76.3% |

Test Example 3

Hygroscopicity Test

(Test Method)

Sample

1.    β-form crystals of DSG           2 g

      Pharmasol® H-15           26 g

The above components are for 20 suppositories.

2.    Lyophilized powder of DSG         2 g

      Pharmasol® H-15           26 g

The above components are for 20 suppositories.

26 g of Pharmasol® H-15 in a beaker was melted by heating on a water bath. After stopping heating, 2 g of β-form crystals of DSG (a lyophilized powder of DSG) was added to the solution at 40°C with stirring, homogenized with enough mixing, filled into suppository molds, cooled at room temperature and solidified by cooling in a refrigerator to give suppositories.

The above suppositories were stored at 25°C at 75% RH, and the increase ratio of weight of the sample was measured.

(Test Results)

Table 4  Hygroscopicity of suppositories

| Sample | Increase ratio of weight (%) | | | |
|---|---|---|---|---|
| | 1w | 2w | 3w | 4w |
| β-form crystals of DSG | 2.0 | 2.7 | 3.6 | 4.4 |
| Lyophilized powder of DSG | 4.8 | 6.2 | 7.4 | 8.4 |

As shown in Table 2, the plasma concentration of DSG obtained by administration of the suppository of the present invention is higher than the concentrations obtained by the oral administration of DSG, by the rectal administration using the aqueous solution of DSG, and by the rectal administration of the suppository of DSG using a hydrophilic base (Macrogol). Particularly, the plasma concentration of DSG obtained by administration of the suppository using the lipophilic base in Example 1 is about 3.5 times at $C_{max}$ higher and about 3 times at $AUC_{0-8h}$ higher than that obtained by administration of Control 1 which exhibited the highest value in the control group.

As shown in Tables 3 and 4, the suppository containing β-form crystals of DSG exhibited a higher DSG stability and a lower hygroscopicity than the suppository containing a lyophilized powder of DSG.

It is apparent that, according to the invention, there is provided a 15-deoxyspergualin suppository which can be obtained high plasma concentration of 15-deoxyspergualin when administered and has good storage stability.

**Claims**

**Claims for the following Contracting States : DE, FR, GB, IT**

1. A suppository which comprises β-form crystals of 15-deoxyspergualin trihydrochloride and a lipophilic base which is solid at an ambient temperature.

2. A suppository according to Claim 1 which comprises 3 - 25 % by weight of β-form crystals of 15-deoxyspergualin and 97 - 75 % by weight of the lipophilic base.

3. A suppository according to Claim 1 which comprises 10 - 20% by weight of β-form crystals of 15-deoxyspergualin and 90 - 80% by weight of the lipophilic base.

4. A suppository according to Claim 1, wherein the lipophilic base which is solid at ambient temperature is a triglyceride of $C_8$ - $C_{18}$ saturated fatty acids containing at least 50% by weight of $C_8$ - $C_{14}$ saturated fatty acids.

5. A suppository according to Claim 4 wherein the $C_8$ - $C_{14}$ saturated fatty acids attached to glycerin are one or more members selected from $C_8$, $C_{10}$, $C_{12}$ and $C_{14}$ saturated fatty acids.

6. A suppository according to Claim 4, wherein the saturated fatty acids attached to glycerin contain an amount of from 32 to 60% by weight of a $C_{12}$ of a $C_{14}$ saturated fatty acid; from 9 to 20% by weight of a $C_{16}$ saturated fatty acid; from 9 to 32% by weight of a $C_{18}$ saturated fatty acid; and 0 to 4% by weight of other fatty acids.

7. A suppository according to Claim 4, wherein the saturated fatty acids attached to glycerin contain an amount of from 44 to 57% by weight of a $C_{12}$ saturated fatty acid; from 17 to 23% by weight of $C_{14}$ saturated fatty acid; from 10 to 14% by weight of a $C_{16}$ saturated fatty acid; from 10 to 22% by weight of a $C_{18}$ saturated fatty acid; and 0 to 2% by weight of other fatty acids.

8. Process for the manufacture of a suppository containing 15-deoxyspergualin trihydrochloride β-form crystals and lipophilic base which is solid at an ambient temperature, comprising
   a) melting by heating one or a mixture of two or more lipophilic bases which are solid at room temperature,
   b) adding 15-deoxyspergualin trihydrochloride β-form crystals and optionnally usual pharmaceutically acceptable additives thereto,
   c) homogenizing the mixture,
   d) filling the mixture into suppository containers or gelatin capsule shells and cooling.

9. Process according to claim 8, wherein the 15-deoxsyspergualin trihydrochloride β-form crystals are obtained by crystallizing 15-deoxyspergualin trihydrochloride in the presence of a mixture of water and a water miscible solvent.

10. Process according to claim 9, wherein the crystallization is carried out by adding a mixture of water and a water miscible solvent to a powder of 15-deoxyspergualin trihydrochloride, thoroughly mixing and allowing the mixture to stand for 3 days at a temperature of 5°C, and removing the resulting β-form crystals by suction filtration.

11. Process according to claim 9 or 10, wherein the mixture of water and a water miscible solvent is 95% ethanol.

12. Process according to claim 11, wherein the ratio of powder of 15-deoxyspergualin trihydrochloride and 95% ethanol is 1 : 5.

13. Process according to one or more of claims 8 to 12, wherein 3 - 25% by wt. of β-form crystals of 15-deoxyspergualin hydrochloride and 97 - 75% by wt. of the lipophilic base are used.

14. Process according to one or more of claims 8 to 13, wherein 10-20% by wt. of β-form crystals of 15-deoxyspergualin hydrochloride and 90 - 80% by wt. of the lipophilic base are used.

**Claims for the following Contracting State : ES**

1. Process for the manufacture of a suppository containing 15-deoxyspergualin trihydrochloride β-form crystals and lipophilic base which is solid at an ambient temperature, comprising
   a) melting by heating one or a mixture of two or more lipophilic bases which are solid at room temperature,
   b) adding 15-deoxyspergualin trihydrochloride β-form crystals and optionally usual pharmaceutically acceptable additives thereto,
   c) homogenizing the mixture,
   d) filling the mixture into suppository containers or gelatin capsule shells and cooling.

2. Process according to claim 1, wherein the 15-deoxsyspergualin trihydrochloride β-form crystals are obtained by crystallizing 15-deoxyspergualin trihydrochloride in the presence of a mixture of water and a water miscible solvent.

3. Process according to claim 2, wherein the crystallization is carried out by adding a mixture of water and a water miscible solvent to a powder of 15-deoxyspergualin trihydrochloride, thoroughly mixing and allowing the mixture to stand for 3 days at a temperature of 5°C, and removing the resulting β-form crystals by suction filtration.

4. Process according to claim 2 or 3, wherein the mixture of water and a water miscible solvent is 95% ethanol.

5. Process according to claim 4, wherein the ration of powder of 15-deoxyspergualin trihydrochloride and 95% ethanal is 1 : 5.

6. Process according to one or more of claims 1 to 5, wherein 3 - 25% by wt. of $\beta$-form crystals of 15-deoxyspergualin hydrochloride and 97 - 75% by wt. of the lipophilic base are used.

7. Process according to one or more of claims 1 to 6, wherein 10-20% by wt. of $\beta$-form crystals of 15-deoxyspergualin hydrochloride and 90 - 80% by wt. of the lipophilic base are used.

8. Process according to one or more of claims 1 to 7, wherein the lipophilic base which is solid at ambient temperature is a triglyceride of $C_8$ to $C_{18}$ saturated fatty acids containing at least 50% by wt. of $C_8$ to $C_{14}$ saturated fatty acids.

9. Process according to claim 8 wherein the $C_8$ - $C_{14}$ saturated fatty acids attached to glycerin are one or more members selected from $C_8$, $C_{10}$, $C_{12}$ und $C_{14}$ saturated fatty acids.

10. Process according to claim 8, wherein the saturated fatty acids attached to glycerin contain an amount of from 32 to 60% by wt. of a $C_{12}$ saturated fatty acid; from 12 to 25% by wt. of a $C_{14}$ saturated fatty acid; from 9 to 20% by wt. of $C_{16}$ saturated fatty acid; from 9 to 32% by wt. of $C_{18}$ saturated fatty acid; and 0 to 4% by wt. of other fatty acids.

11. Process according to claim 8, wherein the saturated fatty acids attached to glycerin contain an amount of from 44 to 57% by wt. a of $C_{12}$ saturated fatty acid; from 17 to 23% by wt. of $C_{14}$ saturated fatty acid; from 10 to 14% by wt. of $C_{16}$ saturated fatty acid; from 10 to 22% by wt. of a $C_{18}$ saturated fatty acid; and 0 to 2% by wt. of other fatty acids.

## Patentansprüche

## Patentansprüche für folgende Vertragsstaaten : DE, FR, GB, IT

1. Suppositorium, das umfaßt (enthält) $\beta$-Form-Kristalle von 15-Deoxyspergualintrihydrochlorid und eine lipophile Basis, die bei Umgebungstemperatur fest ist.

2. Suppositorium nach Anspruch 1, das 3 bis 25 Gew.-% $\beta$-Form-Kristalle von 15-Deoxyspergualin und 97 bis 75 Gew.-% der lipophilen Basis enthält.

3. Suppositorium nach Anspruch 1, das 10 bis 20 Gew.-% $\beta$-Form-Kristalle von 15-Deoxyspergualin und 90 bis 80 Gew.-% der lipophilen Basis enthält.

4. Suppositorium nach Anspruch 1, worin die lipophile Basis, die bei Umgebungstemperatur fest ist, ein Triglycerid von gesättigten $C_8$-$C_{18}$-Fettsäuren ist, die mindestens 50 Gew.-% gesättigte $C_8$-$C_{14}$-Fettsäuren enthalten.

5. Suppositorium nach Anspruch 4, worin die an Glycerin gebundenen gesättigten $C_8$-$C_{14}$-Fettsäuren einen oder mehr Vertreter darstellen, der ausgewählt wird aus der Gruppe der gesättigten $C_8$-, $C_{10}$-, $C_{12}$- und $C_{14}$-Fettsäuren.

6. Suppositorium nach Anspruch 4, worin die an Glycerin gebundenen gesättigten Fettsäuren 32 bis 60 Gew.-% einer gesättigten $C_{12}$-Fettsäure, 12 bis 25 Gew.-% einer gesättigten $C_{14}$-Fettsäure, 9 bis 20 Gew.-% einer gesättigten $C_{16}$-Fettsäure, 9 bis 32 Gew.-% einer gesättigten $C_{18}$-Fettsäure und 0 bis 4 Gew.-% andere Fettsäuren enthalten.

7. Suppositorium nach Anspruch 4, worin die an Glycerin gebundenen gesättigten Fettsäuren 44 bis 57 Gew.-% einer gesättigten $C_{12}$-Fettsäure, 17 bis 22 Gew.-% einer gesättigten $C_{14}$-Fettsäure, 10 bis 14 Gew.-% einer gesättigten $C_{16}$-Fettsäure, 10 bis 22 Gew.-% einer gesättigten $C_{18}$-Fettsäure und 0 bis 2 Gew.-% andere Fettsäuren enthalten.

8. Verfahren zur Herstellung eines Suppositoriums, das 15-Deoxyspergualintrihydrochlorid-$\beta$-Form-Kristalle und eine lipophile Basis enthält, die bei Umgebungstemperatur fest ist, das umfaßt

a) das Schmelzen einer lipophilen Basis oder einer Mischung von zwei oder mehr lipophilen Basen, die bei Raumtemperatur fest sind, durch Erhitzen,
b) die Zugabe von 15-Deoxyspergualintrihydrochlorid-β-Form-Kristallen und gegebenenfalls von üblichen pharmazeutisch akzeptablen Additiven,
c) das Homogenisieren der Mischung und
d) das Einfüllen der Mischung in Suppositorien-Behälter oder Gelatinekapsel-Hüllen und das Abkühlen.

9. Verfahren nach Anspruch 8, worin die 15-Deoxyspergualintrihydrochlorid-β-Form-Kristalle erhalten werden durch Kristallisieren von 15-Deoxyspergualintrihydrochlorid in Gegenwart einer Mischung aus Wasser und einem mit Wasser mischbaren Lösungsmittel.

10. Verfahren nach Anspruch 9, worin die Kristallisation durchgeführt wird durch Zugabe einer Mischung aus Wasser und einem mit Wasser mischbaren Lösungsmittel zu einem Pulver von 15-Deoxyspergualintrihydrochlorid, gründliches Durchmischen und Stehenlassen der Mischung 3 Tage lang bei einer Temperatur von 5°C und Entfernen der resultierenden β-Form-Kristalle durch Absaugen.

11. Verfahren nach Anspruch 9 oder 10, worin die Mischung aus Wasser und einem mit Wasser mischbaren Lösungsmittel 95 %iges Ethanol ist.

12. Verfahren nach Anspruch 11, worin das Verhältnis zwischen dem Pulver von 15-Deoxyspergualintrihydrochlorid und 95 %igem Ethanol 1:5 beträgt.

13. Verfahren nach einem oder mehr der Ansprüche 8 bis 12, worin 3 bis 25 Gew.-% der β-Form-Kristalle von 15-Deoxyspergualinhydrochlorid und 97 bis 75 Gew.-% der lipophilen Basis verwendet werden.

14. Verfahren nach einem oder mehr der Ansprüche 8 bis 13, worin 10 bis 20 Gew.-% der β-Form-Kristalle von 15-Deoxyspergualinhydrochlorid und 90 bis 80 Gew.-% der lipophilen Basis verwendet werden.

**Patentansprüche für folgenden Vertragsstaat : ES**

1. Verfahren zur Herstellung eines Suppositoriums, das 15-Deoxyspergualintrihydrochlorid-β-Form-Kristalle und eine lipophile Basis enthält, die bei Umgebungstemperatur fest ist, das umfaßt
a) das Schmelzen einer lipophilen Basis oder einer Mischung von zwei oder mehr lipophilen Basen, die bei Raumtemperatur fest sind, durch Erhitzen,
b) die Zugabe von 15-Deoxyspergualintrihydrochlorid-β-Form-Kristallen und gegebenenfalls von üblichen pharmazeutisch akzeptablen Additiven,
c) das Homogenisieren der Mischung und
d) das Einfüllen der Mischung in Suppositorien-Behälter oder Gelatinekapsel-Hüllen und das Abkühlen.

2. Verfahren nach Anspruch 1, worin die 15-Deoxyspergualintrihydrochlorid-β-Form-Kristalle erhalten werden durch Kristallisieren von 15-Deoxyspergualintrihydrochlorid in Gegenwart einer Mischung aus Wasser und einem mit Wasser mischbaren Lösungsmittel.

3. Verfahren nach Anspruch 2, worin die Kristallisation durchgeführt wird durch Zugabe einer Mischung aus Wasser und einem mit Wasser mischbaren Lösungsmittel zu einem Pulver von 15-Deoxyspergualintrihydrochlorid, gründliches Durchmischen und Stehenlassen der Mischung 3 Tage lang bei einer Temperatur von 5°C und Entfernen der resultierenden β-Form-Kristalle durch Absaugen.

4. Verfahren nach Anspruch 2 oder 3, worin die Mischung aus Wasser und einem mit Wasser mischbaren Lösungsmittel 95 %iges Ethanol ist.

5. Verfahren nach Anspruch 4, worin das Verhältnis zwischen dem Pulver von 15-Deoxyspergualintrihydrochlorid und 95 %igem Ethanol 1:5 beträgt.

6. Verfahren nach einem oder mehr der Ansprüche 1 bis 5, worin 3 bis 25 Gew.-% der β-Form-Kristalle von 15-Deoxyspergualinhydrochlorid und 97 bis 75 Gew.-% der lipophilen Basis verwendet werden.

7. Verfahren nach einem oder mehr der Ansprüche 1 bis 6, worin 10 bis 20 Gew.-% der β-Form-Kristalle von 15-Deoxyspergualinhydrochlorid und 90 bis 80 Gew.-% der lipophilen Basis verwendet werden.

**8.** Verfahren nach einem oder mehr der Ansprüche 1 bis 7, worin die lipophile Basis, die bei Umgebungstemperatur fest ist, ein Triglycerid von gesättigten $C_8$-$C_{18}$-Fettsäuren ist, die mindestens 50 Gew.-% gesättigte $C_8$-$C_{14}$-Fettsäuren enthalten.

**9.** Verfahren nach Anspruch 8, worin die an Glycerin gebundenen gesättigten $C_8$-$C_{14}$-Fettsäuren ein oder mehr Vertreter sind, die ausgewählt werden aus der Gruppe der gesättigten $C_8$-, $C_{10}$-, $C_{12}$- und $C_{14}$-Fettsäuren.

**10.** Verfahren nach Anspruch 8, worin die an Glycerin gebundenen Fettsäuren 32 bis 60 Gew.-% einer gesättigten $C_{12}$-Fettsäure, 12 bis 25 Gew.-% einer gesättigten $C_{14}$-Fettsäure, 9 bis 20 Gew.-% einer gesättigten $C_{16}$-Fettsäure, 9 bis 32 Gew.-% einer gesättigten $C_{18}$-Fettsäure und 0 bis 4 Gew.-% andere Fettsäuren enthalten.

**11.** Verfahren nach Anspruch 8, worin die an Glycerin gebundenen gesättigten Fettsäuren 44 bis 57 Gew.-% einer gesättigten $C_{12}$-Fettsäure, 17 bis 23 Gew.-% einer gesättigten $C_{14}$-Fettsäure, 10 bis 14 Gew.-% einer gesättigten $C_{16}$-Fettsäure, 10 bis 22 Gew.-% einer gesättigten $C_{18}$-Fettsäure und 0 bis 2 Gew.-% andere Fettsäuren enthalten.

## Revendications

### Revendications pour les Etats contractants suivants : DE, FR, GB, IT

**1.** Suppositoire comprenant des cristaux sous forme β de trichlorhydrate de 15-désoxyspergualine, et un excipient lipophile solide à température ambiante.

**2.** Suppositoire selon la revendication 1, comprenant de 3 à 25 % en poids de cristaux sous forme β de 15-désoxyspergualine et de 97 à 75 % en poids de l'excipient lipophile.

**3.** Suppositoire selon la revendication 1, comprenant de 10 à 20 % en poids de cristaux sous forme β de 15-désoxyspergualine et de 90 à 80 % en poids de l'excipient lipophile.

**4.** Suppositoire selon la revendication 1, dans lequel l'excipient lipophile solide à température ambiante, est un triglycéride dérivé d'acides gras saturés en $C_8$ à $C_{18}$, contenant au moins 50 % en poids d'acides gras saturés en $C_8$ à $C_{14}$.

**5.** Suppositoire selon la revendication 4, dans lequel les acides gras saturés en $C_8$ à $C_{14}$ liés à la glycérine, comprennent au moins un composé choisi parmi les acides gras saturés en $C_8$, $C_{10}$, $C_{12}$ et $C_{14}$.

**6.** Suppositoire selon la revendication 4, dans lequel les acides gras saturés liés à la glycérine, contiennent une quantité de 32 à 60 % en poids d'un acide gras saturé en $C_{12}$ ; de 12 à 25 % en poids d'un acide gras saturé en $C_{14}$ ; de 9 à 20 % en poids d'un acide gras saturé en $C_{16}$ ; de 9 à 32 % d'un acide gras saturé en $C_{18}$ et de 0 à 4 % en poids d'autres acides gras.

**7.** Suppositoire selon la revendication 4, dans lequel les acides gras saturés liés à la glycérine, contiennent une quantité de 44 à 57 % en poids d'un acide gras saturé en $C_{12}$ ; de 17 à 23 % en poids d'un acide gras saturé en $C_{14}$ ; de 10 à 14 % en poids d'un acide gras saturé en $C_{16}$, de 10 à 22 % en poids d'un acide gras saturé en $C_{18}$ ; et de 0 à 2 % en poids d'autres acides gras.

**8.** Procédé de fabrication d'un suppositoire contenant des cristaux sous forme β de trichlorhydrate de 15-désoxyspergualine et un excipient lipophile solide à température ambiante, selon lequel :
a) on fond, en chauffant, un mélange d'au moins deux ou plusieurs excipients lipophiles solides à la température ambiante ;
b) on ajoute des cristaux de trichlorhydrate de 15-désoxyspergualine sous forme β et éventuellement des additifs pharmaceutiquement acceptables habituels ;
c) on homogénéise le mélange ; et
d) on introduit le mélange dans des récipients à suppositoire ou des enveloppes formant capsule de gélatine, et on refroidit.

9. Procédé selon la revendication 8, dans lequel les cristaux sous forme β de trichlorhydrate de 15-désoxyspergualine, sont obtenus par cristallisation du trichlorhydrate de 15-désoxyspergualine en présence d'un mélange d'eau et d'un solvant miscible dans l'eau.

10. Procédé selon la revendication 9, dans lequel la cristallisation est effectuée en ajoutant un mélange d'eau et d'un solvant miscible dans l'eau, dans une poudre de trichlorhydrate de 15-désoxyspergualine, en mélangeant complètement et en laissant le mélange reposer pendant trois jours à une température de 5 °C, et on sépare les cristaux sous forme β résultants, par filtration par aspiration.

11. Procédé selon la revendication 9 ou 10, dans lequel le mélange d'eau et d'un solvant miscible dans l'eau, consiste en de l'éthanol à 95 %.

12. Procédé selon la revendication 11, dans lequel le rapport de la poudre de trichlorhydrate de 15-désoxyspergualine à l'éthanol à 95 % est de 1:5.

13. Procédé selon au moins l'une des revendications 8 à 12, dans lequel on emploie de 3 à 25 % en poids de cristaux sous forme β de trichlorhydrate de 15-désoxyspergualine, et de 97 à 75 % en poids de l'excipient lipophile.

14. Procédé selon au moins l'une des revendications 8 à 13, dans lequel on emploie de 10 à 20 % en poids de cristaux sous forme β de trichlorhydrate de 15-désoxyspergualine, et de 90 à 80 % en poids de l'excipient lipophile.

**Revendications pour l'Etat contractant suivant : ES**

1. Procédé de fabrication d'un suppositoire contenant des cristaux sous forme β de trichlorhydrate de 15-désoxyspergualine et un excipient lipophile solide à température ambiante, selon lequel :
   a) on fond, en chauffant, au moins deux ou plusieurs excipients lipophiles solides à la température ambiante ;
   b) on ajoute des cristaux de trichlorhydrate de 15-désoxyspergualine sous forme β et éventuellement des additifs pharmaceutiquement acceptables habituels ;
   c) on homogénéise le mélange ; et
   d) on introduit le mélange dans des récipients à suppositoire ou des enveloppes formant capsule de gélatine, et on refroidit.

2. Procédé selon la revendication 1, dans lequel les cristaux sous forme β de trichlorhydrate de 15-désoxyspergualine, sont obtenus par cristallisation du trichlorhydrate de 15-désoxyspergualine en présence d'un mélange d'eau et d'un solvant miscible dans l'eau.

3. Procédé selon la revendication 2, dans lequel la cristallisation est effectuée en ajoutant un mélange d'eau et d'un solvant miscible dans l'eau, dans une poudre de trichlorhydrate de 15-désoxyspergualine, en mélangeant complètement et en laissant le mélange reposer pendant trois jours à une température de 5 °C, et on sépare les cristaux sous forme β résultants, par filtration par aspiration.

4. Procédé selon la revendication 2 ou 3, dans lequel le mélange d'eau et d'un solvant miscible dans l'eau, consiste en de l'éthanol à 95 %.

5. Procédé selon la revendication 4, dans lequel le rapport de la poudre de trichlorhydrate de 15-désoxyspergualine à l'éthanol à 95 % est de 1:5.

6. Procédé selon au moins l'une des revendications 1 à 5, dans lequel on emploie de 3 à 25 % en poids de cristaux sous forme β de trichlorhydrate de 15-désoxyspergualine, et de 97 à 75 % en poids de l'excipient lipophile.

7. Procédé selon au moins l'une des revendications 1 à 6, dans lequel on emploie de 10 à 20 % en poids de cristaux sous forme β de trichlorhydrate de 15-désoxyspergualine, et de 90 à 80 % en poids de l'excipient lipophile.

8. Procédé selon au moins une des revendications 1 à 7, dans lequel la base lipophile solide à température ambiante, est un triglycéride dérivé d'acides gras saturés en $C_8$ à $C_{18}$ contenant au moins 50 % en poids d'acides gras en $C_8$ à $C_{14}$.

9. Procédé selon la revendication 8, dans lequel les acides gras saturés en $C_8$ à $C_{14}$ liés à la glycérine, comprennent au moins un composé choisi parmi les acides gras saturés en $C_8$, $C_{10}$, $C_{12}$ et $C_{14}$.

10. Procédé selon la revendication 8, dans lequel les acides gras saturés liés à la glycérine, contiennent une quantité de 32 à 60 % en poids d'un acide gras saturé en $C_{12}$, de 12 à 25 % en poids d'un acide gras saturé en $C_{14}$ ; de 9 à 20 % en poids d'un acide gras saturé en $C_{16}$ ; de 9 à 32 % en poids d'un acide gras saturé en $C_{18}$ et de 0 à 4 % en poids d'autres acides gras.

11. Procédé selon la revendication 8, dans lequel les acides gras saturés liés à la glycérine contiennent une quantité de 44 à 57 % en poids d'un acide gras saturé en $C_{12}$ ; de 17 à 23 % en poids d'un acide gras saturé en $C_{14}$, de 10 à 14 % en poids d'un acide gras saturé en $C_{16}$ ; de 10 à 22 % en poids d'un acide gras saturé en $C_{18}$ et de 0 à 2 % en poids d'autres acides gras.